# EUROPEAN PATENT SPECIFICATION

(11) **EP 1 492 876 B1**
(45) Date of publication and mention of the grant of the patent: **05.12.2007**
(21) Application number: 02716998.6
(22) Date of filing: 26.03.2002
(51) Int. Cl.: C12N 15/88, A61K 47/18, C07D 207/12

(54) **CATIONIC AMPHIPHILES FOR INTRACELLULAR DELIVERY OF THERAPEUTIC MOLECULES, COMPOSITION, PROCESS AND USE THEREOF**
KATONISCHE AMPHIPHILE ZUR INTRAZELLULÄREN VERABREICHUNG THERAPEUTISCHER MOLEKÜLE, ZUSAMMENSETZUNG, VERFAHREN SOWIE DEREN VERWENDUNG
AMPHIPHILES CATIONIQUES PERMETTANT LA LIBERATION INTRACELLULAIRE DE MOLECULES THERAPEUTIQUES, COMPOSITION LES COMPRENANT, PROCEDE ET LEUR UTILISATION

(43) Date of publication of application: 05.01.2005
(73) Proprietor: COUNCIL OF SCIENTIFIC AND INDUSTRIAL RESEARCH, New Delhi 110 001 (IN)
(72) Inventor: KUMAR, Majeti Bharat, Indian Inst. of Chemical T., Andhra Pradesh 500 007 (IN); CHAUDHURI, Arabinda, Indian Inst. of Chemical T., Andhra Pradesh 500 007 (IN); RAMADAS, Yerramsetti, Indian Inst. of Chemical T., Andhra Pradesh 500 007 (IN); RAO, Nalam M., Indian Inst. of Chemical T., Andhra Pradesh 500 007 (IN)
(74) Representative: Portal, Gérard
(86) International application number: PCT/IB2002/001148
(87) International publication number: WO 2003/080847

(56) References cited:
- EP-A- 0 539 114
- EP-A- 0 835 604
- WO-A-01/42200
- WO-A-96/10555
- WO-A-97/46223
- FR-A- 2 802 925
- SK-B- 278 487
- SOLODIN I ET AL: "A NOVEL SERIES OF AMPHIPHILIC IMIDAZOLINIUM COMPOUNDS FOR IN VITRO AND IN VIVO GENE DELIVERY" BIOCHEMISTRY, AMERICAN CHEMICAL SOCIETY. EASTON, PA, US, vol. 34, 1995, pages 13537-13544, XP000602216 ISSN: 0006-2960 cited in the application
- DEVINSKY, FERDINAND ET AL: "Aggregation properties as a measure of lipophilicity in QSAR studies of antimicrobially active amphiphiles" QSAR DES. BIOACT. COMPD. (1992), 233-47. EDITOR(S): KUCHAR, M. PUBLISHER PROUS, BARCELONA, SPAIN., XP001083945
- HENDERSON, LYDIA M. ET AL: "Superoxide generation is inhibited by phospholipase A2 inhibitors. Role for phospholipase A2 in the activation of the NADPH oxidase" BIOCHEM. J. (1989), 264(1), 249-55, XP001087756
- VESELOVSKA, JULIANA ET AL: "Organic ammonium salts. IV. Solubilization properties of ammonium salts derived from heterocycloalkanes" TENSIDE DETERG. (1978), 15(4), 196-9, XP001087708
- CALAS, MICHELE ET AL: "Antimalarial Activity of Compounds Interfering with Plasmodium falciparum Phospholipid Metabolism: Comparison between Mono- and Bisquaternary Ammonium Salts" JOURNAL OF MEDICINAL CHEMISTRY (2000), 43(3), 505-516, XP001066314
- CHEMICAL ABSTRACTS, vol. 111, no. 25, 18 December 1989 (1989-12-18) Columbus, Ohio, US; abstract no. 225296, TSUDA, YOSHINORI ET AL: "Anthelmintics containing N-alkyl- or N-alkanoylpyrrolidines, -piperidines and -morpholines" XP002213009 & JP 01 045302 A (JAPAN) 17 February 1989 (1989-02-17)
- CHEMICAL ABSTRACTS, vol. 111, no. 12, 18 September 1989 (1989-09-18) Columbus, Ohio, US; abstract no. 102569, TSUDA, YOSHINORI ET AL: "Anticariogenic dentifrices containing pyrrolidines" XP002213010 & JP 63 301813 A (NICHIBAI BOEKI K. K., JAPAN) 8 December 1988 (1988-12-08)
- CHEMICAL ABSTRACTS, vol. 109, no. 7, 15 August 1988 (1988-08-15) Columbus, Ohio, US; abstract no. 50259, MATSUDA, HIDEAKI ET AL: "Plant growth regulators containing amino compounds" XP002213011 & JP 63 002904 A (S. S. PHARMACEUTICAL CO., LTD., JAPAN) 7 January 1988 (1988-01-07)
- SKARZEWSKI, JACEK ET AL: "Synthesis of C2 symmetric primary vicinal diamines. Double stereospecific Mitsunobu reaction on the heterocyclic diols derived from tartaric acid" TETRAHEDRON: ASYMMETRY (1997), 8(11), 1861-1867, XP004074672
- CHEMICAL ABSTRACTS, vol. 107, no. 8, 24 August 1987 (1987-08-24) Columbus, Ohio, US; abstract no. 68042, TANAKA, KATSUHIKO ET AL: "Charge-donating materials for electrostatic image development" XP002213012 & JP 61 258269 A (CANON K. K., JAPAN) 15 November 1986 (1986-11-15)

## Description

### TECHNICAL FIELD

The present invention relates to novel cationic amphiphiles containing cyclic head group. The present invention also relates to a pharmaceutical composition comprising said cationic amphiphiles, useful for the delivery of biologically active therapeutic molecules into body cells/tissues of mammals.

### BACKGROUND ART

Although many defective genes associated with numerous genetic diseases have been identified and characterized, because of the selective permeability of biological cell membranes, delivering required amounts of therapeutically important genes into the target body cells is often a daunting challenge.

Thus, success of gene therapy approach in treating genetic diseases depends, in a major way, on the development of efficient and safe gene delivery reagents that will facilitate the intracellular delivery of therapeutic genes into the particular body cells of a patient. Accordingly, development of safe and efficient gene delivery reagents and methods that can facilitate entry of functional genes into body cells are of great medical importance.

Amphiphilic molecules containing both polar and non-polar regions in their molecular architecture have been used in delivering therapeutically important molecules into cells. This makes sense given the existence of both polar and non-polar segments in biological cell membranes. Cationic amphiphiles are the particularly important class of amphiphilic compounds used most extensively for enhancing intracellular delivery of many biologically active therapeutic compounds. Broadly speaking, at physiological pH the polar segment of cationic amphiphile interacts with the therapeutically important molecules including polyanionic macromolecular DNA, RNA, proteins etc. while the non-polar region of the cationic amphiphiles facilitate the passage of the therapeutic compounds through the non-polar part of the cell membranes.

The following references are examples of cationic amphiphiles that are known in the art to be useful for enhancing the intracellular delivery of therapeutically important molecules. In addition to the molecular structures, these prior arts contain useful information and discussion on the properties of the cationic amphiphiles those are believed to be responsible for their carrier properties.

Felgner et al., Proc.Natl.Acad.Sci. U.S.A., 84, 7413-7417 (1987), reported the first use of a highly efficient cationic lipid N-[1-(2,3-dioleyloxy)propyl]-N,N,N-trimethyl ammonium chloride(DOTMA) as the DNA transfection vector.

U.S. Pat.Nos. 4,897,355 and 4,946,787 (1990) reported the synthesis and use of N-[.omega..(.omega.-1)-dialkyloxy]-and N-[..omega..(.omega.-1)-dialkenyloxyl-alk-1-yl-N,N,N-tetrasubstituted ammonium amphiphiles and their pharmaceutical formulation as efficient transfection vectors.

Leventis, R.and Silvius, J.R Biochim. Biophys. Acta. 1023, 124 -132, (1990) reported the interactions of mammalian cells with lipid dispersions containing novel metabolizable cationic amphiphiles.

U.S. Pat.Nos.5,264,618 (1993) reported the synthesis and use of additional series of highly efficient cationic lipids for intracellular delivery of biologically active molecules.

Felgner et al. J.Biol.Chem. 269, 2550-2561 (1994) reported enhanced gene delivery and mechanistic studies with a novel series of cationic lipid formulations.

U.S.Pat.No. 5,283,185 (1994) reported the synthesis and use of 3β[N-(N¹,N¹-dimethylaminoethane)carbamoyl]cholesterol, termed as "DC-Chol"for delivery of a plasmid carrying a gene for chloramphenicol acetyl transferase into cultured mammalian cells.

U.S. Pat. No. 5,283,185 (1994) reported the use of N-[2-[[2,5-bis[(3-aminopropyl)amino]-1-Oxopentyl]aminoethyl]-N,N-dimethyl-2,3-bis-(9-octadecenyloxy)-1-Propanaminium tetra(trifluoroacetate), one of the most widely used cationic lipids in gene delivery. The pharmaceutical formulation containing this cationic lipid is sold commercially under the trade name "Lipofectamine".

Solodin et al. Biochemistry 34,13537-13544, (1995) reported a novel series of amphilic imidazolinium compounds for in vitro and in vivo gene delivery.

Wheeler et al. Proc. Natl. Acad.Sci. U.S.A 93, 11454-11459, (1996) reported a novel cationic lipid that greatly enhances plasmid DNA delivery and expression in mouse lung.

U.S.Pat No. 5,527,928 (1996) reported the synthesis and the use of N,N,N,N-tetramethyl-N,N-bis (hydroxy ethyl)-2,3-di(oleolyoxy)-1,4-butanediammonim iodide i.e pharmaceutical formulation as transfection vector.

U.S.Pat.No. 5.698,721 (1997) reported the synthesis and use of alkyl O-phosphate esters of diacylphosphate compounds such as phosphatidylcholine or posphatidylethanolamine for intracellular delivery of macromolecules.

U.S.Pat.Nos. 5,661,018; 5,686,620and 5,688,958 (1997) disclosed a novel class of cationic phospholipids containing phosphotriester derivatives of phosphoglycerides and sphingolipids efficient in the lipofection of nucleic acids.

U.S.Pat.No. 5,614,503 (1997) reported the synthesis and use of an amphiphatic transporter for delivery of nucleic acid into cells, comprising an essentially nontoxic, biodegradable cationic compound having a cationic polyamine head group capable of binding a nucleic acid and a cholesterol lipid tail capable of associating with a cellular membrane.

U.S.Pat.No. 5,705,693 (1998) disclosed the method of preparation and use of new cationic lipids and intermediates in their synthesis that are useful for transfecting nucleic acids or peptides into prokaryotic or eukaryotic cells. These lipids comprise one or two substituted arginine, lysine or ornithine residues, or derivatives thereof, linked to a lipophilic moiety.

U.S.Pat. No.5, 719,131 (1998) has reported the synthesis of a series of novel cationic amphiphiles that facilitate transport of genes into cells. The amphiphiles contain lipophilic groups derived from steroids, from mono or dialkylamines, alkylamines or polyalkylamines.

US. Patent No. 5,527,928, (1996) reported on the synthesis and transfection biology of a novel cationic lipid namely, N, N, N',N'-tetramethyl-N,N'-bis (2-hydroxyethyl)-2,3-di(oleoylicoxy)-1,4-butaneammonium iodide.

WO01/42200, SOLODIN I ET AL " A novel series of amphiphilic imidazolinium compounds for in vitro and in vivo gene delivery" biochemistry, American Chemical Society., Vol. 34, 1995, pages 13537-13544, WO97 46223, FR 2 802 925, and WO96 10555 relates to cationic amphiphiles comprising an ammonium part.

WO01/42200 is particularly directed to cationic amphiphiles for intracellular delivery of therapeutic molecules. These cationic amphiphiles are based on a glyceryl backbone.

### OTHER PUBLICATIONS

Behr, J.P., Demeneix, B., Loeffler, J.P. and Perex-Mutul, J. Proc. Natl. Acad. Sci. USA, 1989, 86, 124-132.
Levantis, R., and Silvius, J.R. Biochim. Biophys. Acta., 1990, 1023, 124-132.
Gao, X. and Huang, L. Biochim. Biophys. Res. Commun., 1991, 179,280-285.
Akao, T., Nakayama, T., Takeshia, K. and Ito, A., Biochem. Mol. Biol. Int., 1994, 34, 915-920.
Felgner, J. H.; Kumar, R.; Sridhar, C. N.; Wheeler, C. J.; Tsai, Y-J.; Border, R.; Ramsey, P.; Martin, M.; Felgner, P. L. J. Biol. Chem., 1994, 269, 2550-2561.
Wheeler, C.J.; Feigner, P.L.; Tsai, Y.J.; Marshall, J.; Sukhu, L.; Doh, S.G.; Hartikka, J.; Nietupski, J.; Manthorpe. M.; Nichols, M. Proc. Natl. Acad. Sci. USA. 1996, 93, 11454-11459.
Bennett, M. J.; Aberle, A. M.; Balasubramaniam, R. P.; Malone, J. G.; Malone, R. W.; Nantz, M. H. J. Med. Chem. 1997, 40, 4069-4078.
Blessing, T.; Remy, J.-S.; Behr, J.-P.; J. Am. Chem. Soc., 1998, 120, 8519-8520.
Wang, J.; Guo, X.; Xu, Y.; Barron, L.; Szoka, F.C., J. Med. Chem, 1998, 41, 2207- 2215
Lim, Y.; Choi, Y. H.; Park, J. J. Am. Chem. Soc., 1999, 121, 5633-5639.
Lim, Y.; Kim, C.; Kim, K.; Kim, S. W.; Park, J. J. Am. Chem. Soc., 2000, 122, 6524-6525.
Zhu, J.; Munn, R. J.; Nantz, M. H. J. Am. Chem. Soc., 2000, 112, 2645-2646.
Vandenburg, Y. R.; Smith, B. D.; Perez-Payan, N.; Davis, A. P.; J. Am. Chem. Soc., 2000, 122, 3252-3253.
Lynn, D. M.; Langer, R.; J. Am. Chem. Soc., 2000, 122, 10761-10768.
Ferrari, M. E.; Rusalov, D.; Enas, J.; Wheeler, C. J.; Nuc. Acid. Res. 2001, 29, 1539-1548.
Banerjee, R.; Das, P. K.; Srilakshmi, G.V.; Chaudhuri, A.; Rao, N. M. J. Med. Chem.1999, 42, 4292-4299.
Banerjee, R.; Mahidhar, Y. V.; Chaudhuri, A.; Gopal, V.; Rao, N. M. J. Med. Chem. 2001, 44, 4176-4185.
Singh, S. R.; Mukherjee, K.; Banerjee, R.; Chaudhuri, A.; Hait, S. K.; Moulik, S. P.; Ramadas, Y.; Vijayalakshmi, A.; Rao, N. M. Chem. Eur. J. (in press).
Floch, V.; Bolc'h, G. Le.; Gable-Guillaume, C.; Bris, N. Le.; Yaouanc, J-J.; Abbayes, H. Des.; Fe'rec, C.; Cle'ment, J-C. Eur. J. Med Chem., 1998, 33, 923-934.
Solodin, 1.; Brown, C.; Bruno, M.; Chow, C.; Jang, E-H.; Debs, R.; Heath, T. Biochemistry, 1995, 34, 13537-13544.

### OBJECTS OF THE INVENTION

The main object of the invention is to provide novel cationic amphiphilic compounds containing polar cyclic head group.

Another object of the invention is to provide novel cationic amphiphilic compounds, which are useful for delivering therapeutically effective amounts of biologically active molecules into cells/tissues of mammals.

Yet another object of the invention is to provide cationic amphiphilic compounds in which a hydrophobic group is directly linked to the positively charged Nitrogen atom, which is itself in the cyclic ring containing two hydroxyl groups.

Still another object of the invention is to provide novel cationic amphiphilic compounds without any glycerol backbone in their structure.

Another object of the invention is to provide novel therapeutic formulation comprising one or more of the cationic amphiphilic compounds of the invention.

It is a further object of the invention to provide therapeutic formulation useful in gene therapy and delivery of biologically active molecules into cells/tissues of mammals.

### SUMMARY OF THE INVENTION

The present invention relates to novel cationic amphiphilic compounds that facilitate the intracellular delivery of biologically active (therapeutic) molecules. The present invention also relates to pharmaceutical compositions comprising such cationic amphiphiles those are useful for delivering biologically active therapeutic molecules into body cells. The novel cationic lipids of the present invention are particularly useful to combat genetic diseases by non-viral gene therapy.

### DETAILED DESCRIPTION OF THE INVENTION

The present invention provides a cationic amphiphile as represented by general formula (II) 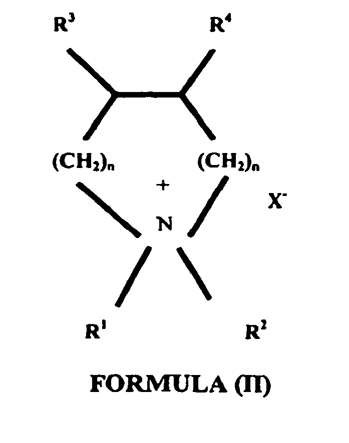
Wherein R₁, R₂, R₃ and R₄ independently or in combination represented as described below:
n=1
R₁ = R₂ = CH₃-(CH₂)₁₀CH₂-, CH₃-(CH₂)₁₂CH₂-, CH₃-(CH₂)₁₄ CH₂-, CH₃-(CH₂)₁₆ CH₂- and/or CH₃-(CH₂)₇-CH=CH(CH₂)₇CH₂.
R₃ = R₄ = hydroxyl group.

An embodiment of the present invention, wherein X is selected from halogen atom, tosyl group and acetate group.

The present invention also provides for a pharmaceutical composition for intracellular delivery of biologically active molecules, said composition comprising:
at least a cationic amphiphile;
   - a bioactive molecule;
   - a colipid; and
   - optionally an additive.

An embodiment of the present invention, a pharmaceutical composition wherein the bioactive cationic amphiphile used to facilitate the intracellular delivery of bioactive molecules having the structural formula (II) as defined above.

Yet another embodiment of the present invention, a pharmaceutical composition wherein the bioactive molecule is selected from the group consisting of ribosomal RNA, antisense polynucleotide of DNA or RNA, polynucleotide of genomic DNA, cDNA or mRNA that encodes for therapeutically important protein, nucleic acid, an oligonucleotide and a peptide.

Still another embodiment of the present invention, a pharmaceutical composition wherein the nucleic acid is circular, lineal plasmid or RNA.

Further embodiment of the present invention, a pharmaceutical composition wherein the colipid is selected from the group consisting of cholesterol, phosphatidylethanolamine, phosphatidylglycerol.

Still another embodiment of the present invention, a pharmaceutical composition according to claim 10 wherein the preferred range of cationic amphiphile and colipid is in the ratio of 1:0-1:2.5.

Yet another embodiment of the present invention, a pharmaceutical composition wherein said composition administered comprises an effective amount of DNA in the range of 0.1-0.5 µg with regard to 50.000 cells of an in vitro system.

Still another embodiment of the present invention, a pharmaceutical composition wherein said composition can be administered intravenously, intramuscularly and intraperitonially.

Yet another embodiment of the present invention, a pharmaceutical composition wherein said additives are selected from physiologically acceptable additives.

Further embodiment of the present invention, a pharmaceutical composition wherein said additives are used to stabilize the formulation and for the effective delivery of bio active molecule.

Still another embodiment of the present invention, a pharmaceutical composition wherein said composition can be formulated with lipophilic therapeutic anti cancer agents selected from doxorubicin, paclitaxel, docetaxel and 5-fluorouracil

Yet another embodiment of the present invention, a pharmaceutical composition wherein said composition is formulated with viral agents selected from Acyclovir.

Still another embodiment of the present invention, a pharmaceutical composition wherein said composition is formulated with antibiotics selected from amphotericin B.

Further embodiment of the present invention, a pharmaceutical composition wherein said composition is formulated with an anti-influenza agent to deliver to the lung, the primary site of the infection.

The present invention also provides a process for the preparation of bioactive cationic amphiphiles of formula (II) said process comprising the steps of:
(a) coupling an appropriate saturated or unsaturated lipophilic aliphatic bromide with saturated or unsaturated lipophilic aliphatic alkyl amine in polar aprotic solvents in presence of base to obtain the corresponding aliphatic hydrophobic secondary amine;
(b) protecting both the terminal primary alcohol groups of an aliphatic alcohols containing additional polar functionalities with suitable protecting groups in polar aprotic solvent;
(c) reacting the secondary amine obtained in step (a) with the primary hydroxyl protected aliphatic polar intermediate obtained in step (b) in polar aprotic solvents in presence of an organic base to obtain quaternized amphiphile compound;
(d) passing the quaternized amphiphilic compound obtained in step (c) through anion-exchange column chromatography and eluting with a mixture of polar organic solvent to obtain the required cationic amphiphile.

An embodiment of the present invention, a process wherein the aliphatic saturated alkyl bromide is selected from the group consisting of 10-30 carbon atoms.

Yet another embodiment of the present invention, a process wherein the aliphatic saturated alkyl amine is selected from the group consisting of 10-30 carbon atoms.

Further embodiment of the present invention, a process wherein the substituted primary hydroxyl group as claimed in step (c) is selected from the group consisting of hydroxyl, hydroxy alkyl, amino or a primary amino and most preferably hydroxyl or amino.

Further embodiment of the present invention, a process wherein the amino group is protected with t-boc, f-moc or any other suitable protective agents.

Still another embodiment of the present invention, a process wherein the aliphatic unsaturated alkyl amine used is selected from the group consisting of 10-30 carbon atoms.

Yet another embodiment of the present invention, a process wherein the polar aprotic solvents in which the reaction is carried out is selected from the group comprising dimethyl formamide, dimethylsulphoxide, pyridine, triethyl amine.

Further embodiment of the present invention, a process wherein the reaction is carried out in the presence of weak base selected from inorganic alkali metal carbonates.

Yet another embodiment of the present invention, a process wherein the primary alcohol protection group is selected from tosyl chloride, mesyl chloride, and the like.

Still another embodiment of the present invention, a process wherein the polar aprotic solvent used in step (a) is selected from dimethyl sulphoxide, N,N-dimethyl formamide, ethyl acetate, tetrahydrofuran and the like.

Yet another embodiment of the present invention, a process wherein the reaction of step (a) is carried out at a temperature between 50° C to 100° C.

Still another embodiment of the present invention, a process wherein the protection of terminal primary hydroxyl groups of step (b) is carried out at a temperature between -10° C to 50°C.

Further embodiment of the present invention, a process wherein primary alcohol is selected from erythrytol or its homologues.

Yet another embodiment of the present invention, a process wherein the polar aprotic solvent used in step (c) is selected from dimethyl sulphoxide, N,N-dimethyl formamide, ethyl acetate, tetrahydrofuran.

Further embodiment of the present invention a process wherein the organic base used in step (c) is selected from triethyl amine, pyridine, piperidine.

Yet another embodiment of the present invention, a process wherein the reaction of step (c) is carried out at a temperature between 50°C to 100°C.

Still another embodiment of the present invention, a process wherein the anion-exchange resin used in step (d) is selected from resin having chloride or bromide ion available for exchange.

Yet another embodiment of the present invention, a process wherein the organic solvent used as ingredients of the polar eluent in step (d) is selected from methanol, ethanol, chloroform, dichloro methane, ethyl acetate.

The present invention further provides Use of a cationic amphiphile represented by a general formula (I) 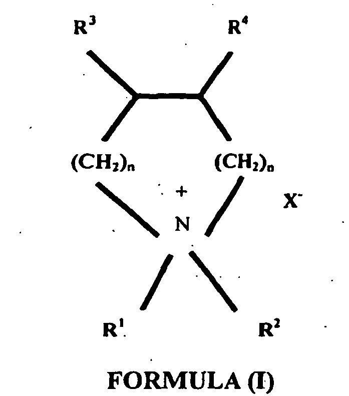 wherein;
R¹ and R² independently represent hydrogen atom or a lipophilic moiety represented by a C₈₋₂₂ saturated alkyl group or an unsaturated alkyl group having one to three unsaturation,
excluding the possible definition for R¹ and R² being simultaneously hydrogen atoms;
R³ and R⁴ are independently represented by any combination of groups selected from hydrogen, hydroxy, hydroxy alkyl, and amino or primary amine ;
n = 1, 2 or 3;
X = an inorganic or organic anion,
for manufacturing a pharmaceutical composition for intracellular delivery of biologically active molecule for administering to a subject said biologically active amphiphile.

Still another embodiment of the present invention, a use wherein R¹ is C₈₋₂₂ alkyl group saturated or unsaturated alkyl group having one to three unsaturation and R² is hydrogen atom or C₈₋₂₂ saturated or unsaturated alkyl group having one to three double unsaturation.

Yet another embodiment of the present invention, a use wherein hydroxy alkyl and primary amine consists of 1-5-carbon atoms.

Still another embodiment of the present invention, a use wherein R¹ independently represents a hydrogen atom and R² is represented by C₈₋₂₂ carbon atoms selected from saturated alkyl or unsaturated alkyl chain having one'to three double bonds.

An embodiment of the present invention, a use wherein R¹ represents C₈₋₂₂ carbon atoms selected from saturated alkyl or unsaturated alkyl chain having one to three double bonds and R2 independently represents a hydrogen atom.

An embodiment of the present invention, a use wherein both R¹ and R² are represented by C₈₋₂₂ carbon atoms selected from saturated alkyl or unsaturated alkyl chain having one to three double bonds.

An embodiment of the present invention, a use wherein the subject is selected from humans and other species including murine, feline, bovine, equine and ovine or non-human primate species.

Another embodiment of the present invention, a use wherein said use is used to combat genetic diseases by non-viral gene therapy.

Still another embodiment of the present invention, a use wherein said composition can be administered intravenously, intramuscularly and intraperitonially.

Yet another embodiment of the present invention, a use wherein cytotoxicities are minimal and the cell viability more than 80%.

Yet another embodiment of the present invention, wherein said use is used to construct cell lines for gene therapy applications in said subjects.

The present invention is also further explained in the form of preferred embodiments.

The distinctive novel structural features common to the cationic amphiphiles disclosed in the present invention include:
(1) The presence of hydrophobic groups which are directly linked to the positively charged nitrogen atom;
(2) The positively charged nitrogen atom is part of the cyclic head group; and
(3) Unlike many other commercially available glycerol-backbone based cationic amphiphiles used in delivering genes into cells, the presently disclosed cationic lipids do not have any glycerol-backbone in their molecular architectures. It is believed that these unique structural features contribute significantly to the increased gene delivery efficiencies of the cationic amphiphiles disclosed herein.

According to the practice of the present invention, "cationic" means the positive charge is either on quaternized nitrogen and/or on a protonated substituent of the cyclic head group. The cationic characters of the present amphiphiles may contribute to the enhanced interaction of the amphiphiles with biologically active molecules such as nucleic acids and/or with cell constituents such as plasma membrane glycoproteins. Such enhanced interaction between the cationic amphiphiles and therapeutically active biological macromolecules and/or cell membrane constituents may play a key role in successfully transporting the therapeutic molecules into the cells.

The invention cationic lipids have a lipophilic domain that facilitates the formation of lipid complexes or aggregates in aqueous solutions. The lipophilicity of the hydrophobic domains and the hydrophilicity of the polar head group domains are such that when the cationic lipids are confronted with aqueous solutions, lipid aggregates are formed in the presence or absence of a second compound. Exemplary lipophilic R₁ and R₂ groups include (1) saturated C₈-C₂₂ alkyl groups and (2) unsaturated C₈-C₂₂ alkyl groups containing one to three unsaturation.

In one preferred embodiment of the presently disclosed cationic lipids where n = 1, both R₁ and R₂ are selected from C₈-C₂₂ saturated alkyl groups, both R₃ and R₄ are independently represented in any combination of groups selected from hydrogen, hydroxyl, hydroxy alkyl, amino or primary amine and X⁻ is selected from chloride or bromide ions.

### Syntheses of the cationic lipids

Scheme I outlines the synthetic strategy employed for preparing the cationic lipids of the present invention containing a five member cyclic head groups (n = 1 in generic Structure of formula (I). The same synthetic scheme is employed to synthesize cationic lipids with seven and nine member cyclic head groups (n = 2 and 3 respectively, in generic Structure of formula (I).

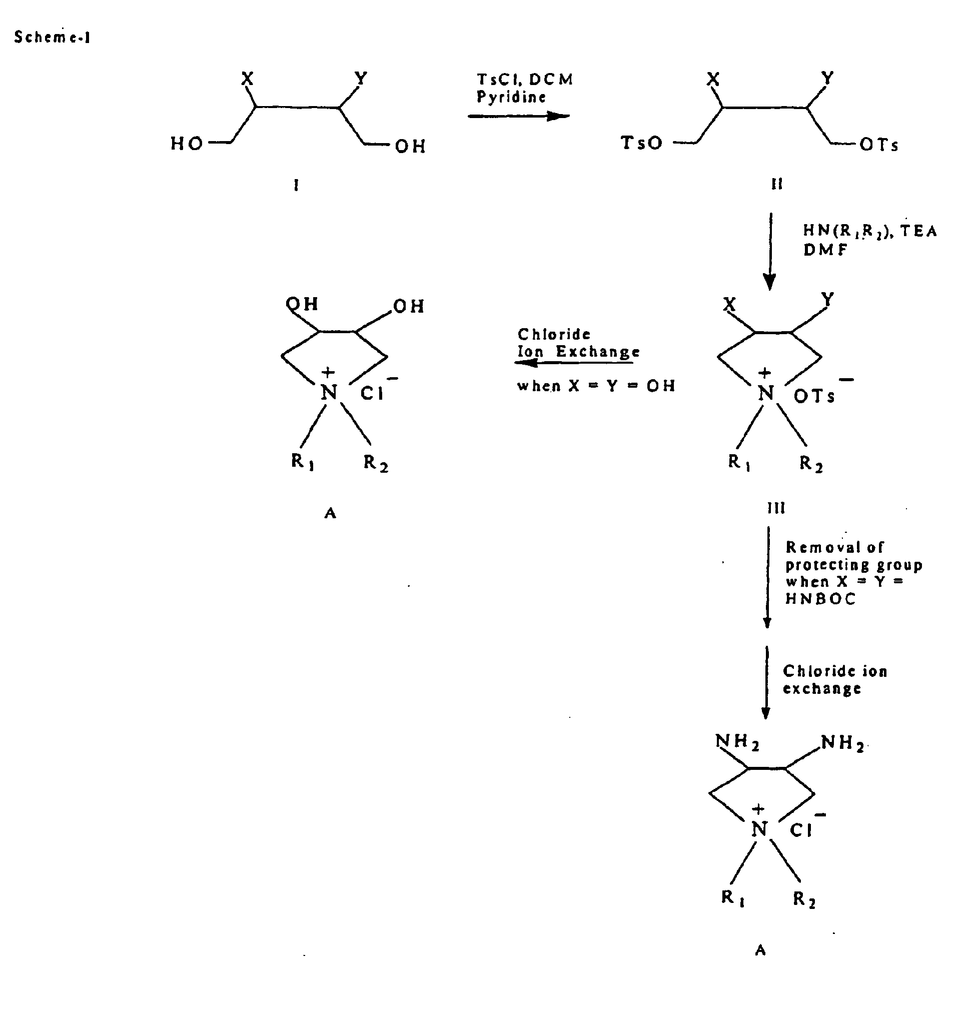

Both the primary hydroxyl groups of the starting alcohol is first converted to their tosyl derivative by reacting them with p-toluenesulfonyl chloride in presence of pyridine as a base. The resulting di-tosyl derivative (the intermediate II in Scheme I) is then subjected to reaction with the appropriate secondary amine in polar aprotic solvent like N.N-dimethyl formamide to form the cationic amphiphile (III), a tosylate counterion containing analog of the present cationic lipids. A final treatment of the intermediate III with chloride ion-exchange resins affords the target cationic amphiphile (I) of the present invention. As shown in Scheme I, to synthesize cationic lipids with two hydroxyl functionalities in the cyclic head group, protecting the secondary hydroxyl group of the starting material is not necessary. However, to prepare cationic amphiphiles with two amino functionalities in the cyclic head group, execution of Scheme I requires protection of both the primary amino group of the starting material and a later deprotection step.

### Formulations

The invention also provides novel therapeutic formulation comprising therapeutically effective amounts of the cationic amphiphilic compounds disclosed herein, biologically active molecules and co-lipids. One or more additional physiologically acceptable substances may be included in the pharmaceutical formulation of the invention to stabilize the formulation for storage or to facilitate successful intracellular delivery of the biologically active molecules. Co-lipids according to the practice of the present invention are useful in mixing with one or more cationic amphiphiles. Cholesterol is an excellent co-lipid for use in combination with the presently described cationic lipids to facilitate successful intracellular delivery of the biologically active molecules. A preferred range of molar ratio of cationic amphiphile to co-lipid is 1:0 to 1:2.5. As such, it is within the art to vary the said range to a considerably wide extent.

Biologically active molecules that can be administered intracellularly in therapeutic amounts using the cationic amphiphilic compound of the present invention include ribosomal RNA, antisense polynucleotide of RNA or DNA, polynucleotide of genomic DNA, cDNA or mRNA that encodes for a therapeutically important protein. The cationic amphiphiles of the present invention may be blended such that one or more of the representatives thereof are used in a combination to facilitate entry of the said biologically active molecules into cells/tissues.

According to the present invention, the amphiphiles are used either in pure form or in combination with other lipids or helper lipids such as cholesterol, phosphatidylethanolamine, phosphatidylglycerol, etc. The said therapeutic formulation can be stored at 0°C-4°C until complexed with the biologically active therapeutic molecules. Agents that prevent bacterial growth and increase the shelf life may be included along with reagents that stabilize the preparation, e.g., low concentrations of glycerol. It is specifically warned that freezing and thawing cycles could cause loss in efficiency of the formulation.

The present invention also provides for various formulations that facilitate intracellular delivery of biologically active molecules.

The present invention also provides for a formulation of cationic amphiphiles and nucleic acid may be administered intravenously besides other routes such as intramuscular and intra peritonial. Further, the said formulation of amphiphiles may be administered to cells at a ratio of 0.1-0.5 microgram of DNA to 50,000 cells in an in vitro system. The amount of amphiphile could be varied from a lipid to DNA charge ratio of 0.1 to 10, considering one positive charge for one amphiphile molecule to one negative charge of a nucleotide base.

The plasmid used is a construct of an Cyto Megalo Virus promoter linked to a reporter gene β-galactosidase as supplied by Gibco BRL Life Technologies, USA (cat no. 10586-014)The plasmid could be of any construction and the example given is merely to demonstrate the efficiency of the amphiphilic formulation. Similar examples of plasmid include PGL-2 and PGL-3 of Promega and others.

The invention further provides a process for the preparation of the said formulation comprising the steps of preparing a dispersion of a cationic amphiphile disclosed in the present invention; contacting said dispersion with a biologically active molecule to form a complex between said amphiphiles and said molecules and contacting cells with said complex thereby facilitating transfer of said biologically active molecules into the cells.

### Cellular Cytotoxicities of the Amphiphiles Disclosed in the Invention

The viabilities of cells in presence of various cationic amphiphiles disclosed herein were checked according to the standard protocol described in "Animal Cell Culture, 2^{nd} Edition. Ed. I. R. L. Press, Oxford University Press (1977)". The transfection efficiencies of the cationic lipids were studied in the range of 0-10 nmole and within this limit, the cell cytotoxicities were observed to be minimal and the cell viabilities were determined to be more than 80%. The cationic amphiphiles were used with varying mole ratios of lipid to DNA using cholesterol as the neutral co-lipid.

### Applications

The process of the present invention can be exploited for preparing cationic transfection lipids with polar cyclic head groups. The invention lipids are useful for delivering polyanions, polypeptides or nucleopolymers into cells. The cationic lipids disclosed herein can be used to deliver an expression vector into a cell for manufacturing or therapeutic use. The expression vectors can be used in gene therapy protocols to deliver a therapeutically useful protein to a cell or for delivering nucleic acids encoding therapeutically useful protein molecules that can generate an immune response in a host for vaccine or other immunomodulatory purposes according to the known uses. The vector-transformed cell can be used to produce commercially useful cell lines, such as a cell line for producing therapeutic proteins or enzymes (e.g. erythropoietin), growth factors (e.g. human growth hormone, G-CSF or interleukins) or other proteins.

The invention lipid-nucleic acid complexes can be used to construct cell lines for gene therapy applications in subjects such as humans or other species including murine, feline, bovine, equine, ovine or non-human primate species. The invention lipids can be used in presence of serum and will thus deliver polyanions into cells in tissue culture medium containing serum in vitro or in animal in vivo.

The invention lipids complexed with nucleopolymers can be used in antisense inhibition of gene expression in a cell by delivering an antisense oligonucleotide into the cell. A cell that is blocked for expression of a specific gene(s) is useful for manufacturing and therapeutic applications. Exemplary manufacturing uses include inhibiting protease synthesis in a cell to increase production (i.e., reduce target protein degradation caused by the protease) of a protein for a therapeutic or diagnostic application. Exemplary therapeutic applications include inhibiting synthesis of cell surface antigens (histocompatibility antigens, such as MHC class II genes, and the like) to reduce rejection and/or to induce immunologic tolerance of the cell after it is implanted into a subject or when the cell is transfected in vivo.

The invention lipids can be formulated with anionic, zwitterionic and lipophilic therapeutic agents including anticancer agents such as doxorubicin, a lipophilic compound, to obtain complexes comprising the invention lipids and a therapeutic agent(s). The invention lipids can be formulated with known antiviral agents such as HPMPC (9-(3-hydroxy-2-phosphonylmethoxy)propyl)cytosine), PMEA(9-(2-phosphonylmethoxy)ethyl)adenine), PMEG PMEA (9-(2-phosphonylmethoxy) ethyl) guanine), PMPA (9-(2-phosphonylmethoxy)propyl)adenine), AZT, 3TC, and their derivatives to obtain lipid complexes with antiviral agents. The invention lipids can be formulated with polyene antibiotics such as amphotericin B. Such formulations are useful for delivering the therapeutic agents into the cytoplasm of cells in vitro or in vivo. Complexes consisting of an invention cationic lipid and an anti-influenza agent can be used to deliver the antiviral agent to the lung, the primary site of infection. These complexes can be prepared by any of the techniques now known or subsequently developed for preparing lipid complexes containing therapeutic agents.

### Brief Description of the Accompanying Drawings

**Figure 1** summarizes the in vitro gene delivery efficiencies for the cationic amphiphiles **1-5** disclosed in the present invention and that of DMRIE, one of the most widely used commercially available transfection lipids under certain conditions and
**Figure 2** shows some representative lipid:DNA interactions for cationic amphiphiles **1-5** disclosed in the present invention.
**Figure 3** provides the in vitro cellular toxicity data of the cationic amphiphiles **1-5** disclosed in the present invention.

### EXAMPLES

The following examples are given by way of illustration of the present invention. Synthetic procedures for representative cationic amphiphiles 1-5 disclosed in the present invention are described in the following examples 1-5. The structures of cationic lipids 1-5 are shown below.

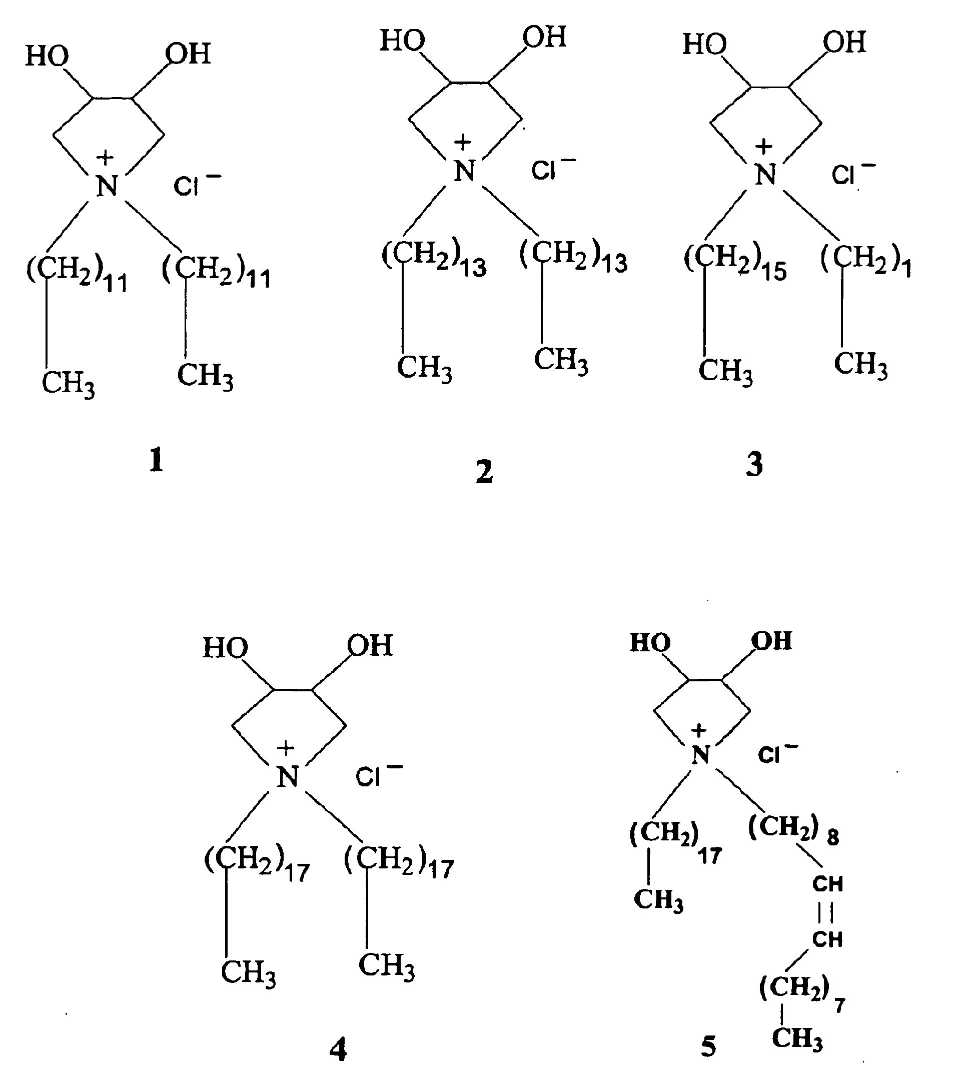

### Example 1:

### Synthesis of N,N-di-n-dodecyl-3,4-dihydroxy pyrrolidinium chloride (amphiphile 1)

Step (a). To 5 g n-dodecyl amine (27 mmol) dissolved in 10 ml DMSO, 6.7 g of n-dodecyl bromide (27 mmol),and 3.7 g of potassium carbonate (27 mmol) were added. The mixture was kept under stirring for 24 hours at 80° C. The reaction mixture was taken in chloroform 100 ml and washed with water (2x 150 ml), the chloroform layer was dried over anhydrous sodium sulfate and filtered. Chloroform was removed from the filtrate on a rotary evaporator and column chromatographic purification (using 60-120 mesh size silica) of the residue using 1-2% methanol in chloroform as an eluent afforded (3.4g, yield 35%) of the desired intermediate secondary amine, namely, N,N-di-n-dodecylamine.

Step (b). To erythritol (4 g 33.9 mmol) dissolved in 100 ml dry pyridine, tosyl chloride (11.6 g, 61 mmol), a few crystals of DMAP were added. The mixture was kept under stirring for 1 h at -5°C. Pyridine was removed on a rotary evaporator and column chromatographic purification (using 60-120 mesh size silica) of the residue using 25-30% ethyl acetate in pet-ether (v/v) as the eluent provided of crude title compound 7 g. Pure ditosylate intermediate was obtained (4 g, 27.4% yield) by final recrystallization of the crude product from chloroform : pet-ether (4:6, 25ml).

Step (c). Reacting the purified ditosylate (0.5 g, 1.2 mmol) with 5 equivalent of secondary amine of step (a) (2.0 g, 5.8 mmol) in triethyl amine 40 ml at 80°C. for 72 hours. Silica gel column chromatographic purification was performed using 60-120 mesh size silica and 10-15% methanol in chloroform as the eluent. Crude title amphiphile No.1 was obtained by subjecting the quaternized salt to repeated treatment (3 times) with chloride ion-exchange resin, each time using a freshly generated Amberlyst A-26 chloride ion exchange column and about methanol 75 ml as the eluent. Finally, pure quaternized title amphiphile salt **1** (0.08 g, 14.5% yield) was obtained by crystallizing the crude product using chloroform : pet-ether 10 ml 3:7. All the isolated intermediates gave spectroscopic data in agreement with their assigned structures.
**¹H-NMR of amphiphile 1** (200 MHz, CDCl₃):δ/ppm = 0.9 [6H, t, 2x-CH₃]; 1.20-1.45 [36H, m, 18x- CH₂]; 1.5-1.8 [4H, m, 2x- CH₂]; 3.4-3.6 [8H, m, -N- CH₂]; 4.05-4.2 [2H, m, - CH-OH]; 4.75-4.8 [1H, m, -CH-OH]; 5.5-5.6 [1H, m, -CH-OH].

### Example 2:

### Procedure for the preparation of N,N-di-n-tetradecyl-3,4-dihydroxy pyrrolidinium chloride (amphiphile No. 2).

Step (a). To 5 g n-tetradecyl amine (23.4 mmol) dissolved in 10 ml DMSO, 6.4 g of n-tetradecyl bromide (23.4 mmol),and 3.2 g of potassium carbonate (23.4 mmol) were added. The mixture was kept under stirring for 24 hours at 80° C. The reaction mixture was taken in 100 ml chloroform and washed with water (2x 150 ml), the chloroform layer was dried over anhydrous sodium sulfate and filtered. Chloroform was removed from the filtrate on a rotary evaporator and column chromatographic purification (using 60-120 mesh size silica) of the residue using 1-2%methanol in chloroform as the eluent afforded 3.4 g (35% yield) of the desired intermediate secondary amine, namely, N,N-di-n-tetradecylamine.

Step (b). To 4 g of erythritol (33.9 mmol) dissolved in 100 ml dry pyridine, tosyl chloride 11.6 g, (61 mmol), a few crystals of DMAP were added. The mixture was kept under stirring for 1 h at -5°C. Pyridine was removed on a rotary evaporator and column chromatographic purification (using 60-120 mesh size silica) of the residue using 25-30% ethyl acetate in pet-ether (v/v) as the eluent provided 7 g of crude title compound. Pure ditosylate intermediate was obtained (4 g, 27.4% yield) by final recrystallization of the crude product from 25 ml 4:6 (v/v) chloroform : pet-ether.

Step (c). Reacting the purified ditosylate (0.5 g, 1.2 mmol) with 5 equivalent of secondary amine (2.4 g, 5.8 mmol) in 40 ml triethyl amine at 80° C. for 72 hours. Silica gel column chromatographic purification was performed using 60-120 mesh size silica and 10-15% methanol in chloroform as the eluent. Crude title amphiphile No.1 was obtained by subjecting the quaternized salt to repeated (3 times) chloride ion-exchange column chromatography, each time using a freshly generated Amberlyst A-26 chloride ion exchange column and about 75 ml of methanol as the eluent. Finally, pure quaternized title amphiphile salt 2 (0.06 g, 9.7% yield) was obtained by crystallizing the crude product using 10 ml 3:7 (v/v) chloroform : pet-ether. All the isolated intermediates gave spectroscopic data in agreement with their assigned structures.
**¹H-NMR of amphiphile 2** (200 MHz, CDCl₃):δ/ppm = 0.9 [6H, t, 2x- CH₃]; 1.20-1.45 [48H, m, 24x- CH_{2]}; 3.4-3.75 [8H, m, 4xN- CH₂]; 4.05-4.20 [2H, m, 2x- CH-OH]; 4.75-4.80 [1H, m, -CH-OH]; 5.5-5.6 [1H, m, -CH-OH].

### Example 3:

### Procedure for the preparation of N,N-di-n-hexadecyl-3,4-dihydroxy pyrrolidinium chloride (amphiphile No. 3).

Step (a). To 5 g n-hexadecyl amine (20.7 mmol) dissolved in 10 ml DMSO, 6.3 g of n-hexadecyl bromide (20.7 mmol),and 2.9 g of potassium carbonate (20.7 mmol) were added. The mixture was kept under stirring for 24 hours at 80° C. The reaction mixture was taken in 100 ml chloroform and washed with water (2x 150 ml), the chloroform layer was dried over anhydrous sodium sulfate and filtered. Chloroform was removed from the filtrate on a rotary evaporator and column chromatographic purification (using 60-120 mesh size silica) of the residue using 1-2%methanol in chloroform as the eluent afforded 3.4 g (35% yield) of the desired intermediate secondary amine, namely, N,N-di-n-hexadecylamine.

Step (b). To 4 g of erythritol (33.9 mmol) dissolved in 100 ml dry pyridine, tosyl chloride 11.6 g, (61 mmol), a few crystals of DMAP were added. The mixture was kept under stirring for 1 h at -5°C. Pyridine was removed on a rotary evaporator and column chromatographic purification (using 60-120 mesh size silica) of the residue using 25-30% ethyl acetate in pet-ether (v/v) as the eluent provided 7 g of crude title compound. Pure ditosylate intermediate was obtained (4 g, 27.4% yield) by final recrystallization of the crude product from 25 ml 4:6 (v/v) chloroform : pet-ether.

Step (c). Reacting the purified ditosylate (0.5 g, 1.2 mmol) with 5 equivalent of secondary amine (2.7 g, 5.8 mmol) in 40 ml triethyl amine at 80° C. for 72 hours. Silica gel column chromatographic purification was performed using 60-120 mesh size silica and 10-15% methanol in chloroform as the eluent. Crude title amphiphile No.1 was obtained by subjecting the quaternized salt to repeated (3 times) chloride ion-exchange column chromatography, each time using a freshly generated Amberlyst A-26 chloride ion exchange column and about 75 ml of methanol as the eluent. Finally, pure quaternized title amphiphile salt 3 (0.13 g, 17.3% yield) was obtained by crystallizing the crude product using 10 ml 3:7 (v/v) chloroform : pet-ether. All the isolated intermediates gave spectroscopic data in agreement with their assigned structures.
**¹H-NMR of amphiphile 3** (200 MHz, CDCl₃):δ/ppm =0.90 [6H, t, 2x-CH₃]; 1.20-1.80 [56H, m, 28x- CH₂]; 3.40-3.75 [8H, m4x-N- CH₂]; 4.05-4.20 [2H, m, 2x- CH-OH]; 4.75-4.80 [1 H, m, -CH- OH]; 5.5-5.6 [1H, m, -CH-OH].

### Example 4:

### Procedure for the preparation of N,N-di-n-octadecyl-3,4-dihydroxy pyrrolidinium chloride (amphiphile No. 4).

Step (a). To 5 g n-octadecyl amine (18.5 mmol) dissolved in 10 ml DMSO, 6.2 g of n-octadecyl bromide (18.5 mmol),and 2.6 g of potassium carbonate (18.5 mmol) were added. The mixture was kept under stirring for 24 hours at 80° C. The reaction mixture was taken in 100 ml chloroform and washed with water (2x 150 ml), the chloroform layer was dried over anhydrous sodium sulfate and filtered. Chloroform was removed from the filtrate on a rotary evaporator and column chromatographic purification (using 60-120 mesh size silica) of the residue using 1-2%methanol in chloroform as the eluent afforded 3.6 g (37% yield) of the desired intermediate secondary amine, namely, N,N-di-n-octadecylamine.

Step (b). To 4 g of erythritol (33.9 mmol) dissolved in 100 ml dry pyridine, tosyl chloride 11.6 g, (61 mmol), a few crystals of DMAP were added. The mixture was kept under stirring for 1 h at -5°C. Pyridine was removed on a rotary evaporator and column chromatographic purification (using 60-120 mesh size silica) of the residue using 25-30% ethyl acetate in pet-ether (v/v) as the eluent provided 7 g of crude title compound. Pure ditosylate intermediate was obtained (4 g, 27.4% yield) by final recrystallization of the crude product from 25 ml 4:6 (v/v) chloroform : pet-ether.

Step (c). Reacting the purified ditosylate (0.5 g, 1.2 mmol) with 5 equivalent of secondary amine (3.0 g, 5.8 mmol) in 40 ml triethyl amine at 80° C. for 72 hours. Silica gel column chromatographic purification was performed using 60-120 mesh size silica and 10-15% methanol in chloroform as the eluent. Crude title amphiphile No.4 was obtained by subjecting the quaternized salt to repeated (3 times) chloride ion-exchange column chromatography, each time using a freshly generated Amberlyst A-26 chloride ion exchange column and about 75 ml of methanol as the eluent. Finally, pure quaternized title amphiphile salt 4 (0.08 g, 10.7% yield) was obtained by crystallizing the crude product using 10 ml 3:7 (v/v) chloroform : pet-ether. All the isolated intermediates gave spectroscopic data in agreement with their assigned structures .
**¹H-NMR of amphiphile 4** (200 MHz, CDCl₃):δ/ppm = 0.90 [6H, 5, 2x- CH₃]; 1.20-1.45 [64H, m, 32x- CH₂]; 3.40-3.75 [8H, m, 4x-N- CH₂]; 4.05-4.20 [2H, m, 2x- CH-OH]; 4.75-4.8 [1H, m, -CH- OH]; 5.5-5.6 [1H, m, -CH-OH].

### Example 5:

### Procedure for the preparation of N-n-octadecyl-N-oleyl-3,4 dihydroxy pyrrolidinium chloride (amphiphile No. 5).

Step (a). To 5 g oleyl amine (18.7 mmol) dissolved in 10 ml DMSO, 6.2 g of octadecyl bromide (18.7 mmol),and 2.6 g of potassium carbonate (18.7 mmol) were added. The mixture was kept under stirring for 24 hours at 80° C. The reaction mixture was taken in 100 ml chloroform and washed with water (2x 150 ml), the chloroform layer was dried over anhydrous sodium sulfate and filtered. Chloroform was removed from the filtrate on a rotary evaporator and column chromatographic purification (using 60-120 mesh size silica) of the residue using 1-2%methanol in chloroform as the eluent afforded 2.7 g (27.8% yield) of the desired intermediate secondary amine, namely, N-n-octadecyl-N-oleyl amine.

Step (b). To 4 g of erythritol (33.9 mmol) dissolved in 100 ml dry pyridine, tosyl chloride 11.6 g, (61 mmol), a few crystals of DMAP were added. The mixture was kept under stirring for 1 h at -5°C. Pyridine was removed on a rotary evaporator and column chromatographic purification (using 60-120 mesh size silica) of the residue using 25-30% ethyl acetate in pet-ether (v/v) as the eluent provided 7 g of crude title compound. Pure ditosylate intermediate was obtained (4 g, 27.4% yield) by final recrystallization of the crude product from 25 ml 4:6 (v/v) chloroform : pet-ether.

Step (c). Reacting the purified ditosylate (0.5 g, 1.2 mmol) with 4 equivalent of secondary amine (2.4 g, 4.7 mmol) in 40 ml triethyl amine at 80° C. for 72 hours. Silica gel column chromatographic purification was performed using 60-120 mesh size silica and 10-15% methanol in chloroform as the eluent. Crude title amphiphile No.4 was obtained by subjecting the quaternized salt to repeated (3 times) chloride ion-exchange column chromatography, each time using a freshly generated Amberlyst A-26 chloride ion exchange column and about 75 ml of methanol as the eluent. Finally, pure quaternized title amphiphile salt 5 (0.05 g, 7.1% yield) was obtained by crystallizing the crude product using 10 ml 3:7 (v/v) chloroform : pet-ether. All the isolated intermediates gave spectroscopic data in agreement with their assigned structures.
**¹H-NMR of amphiphile 5** (200 MHz, CDCl₃):δ/ppm = 0.90 [6H, t, x2- CH₃]; 1.20-1.45 [56H, m, 28x- CH₂]; 1.90-2.00 [4H, m, 2x-CH=CH-CH₂]; 3.40-3.75 [8H, m, 4x-N- CH₂]; 4.05-4.20 [2H, m, -CH-OH]; 4.75-4.80 [1H, m, -CH-OH]; 5.20-5.40 [1H, m, -CH-OH].

### Example 6:

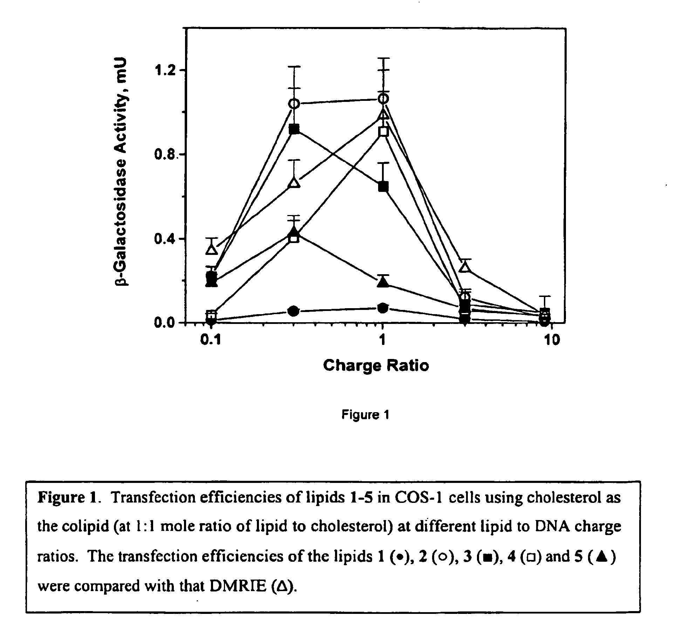

**Cell Transfection.** COS-1 cells were seeded at a density of 15,000 cells/well in a 96-well plate eighteen hours before the transfection. 0.15 µg of plasmid DNA was complexed with varying amount of lipid (0.05 - 4.3 nmoles) in 13 µl of plain DMEM medium for 30 min. The charge ratios were varied from 0.1 : 1 to 9 : 1 (+/-) over this range of the lipid. The complex was diluted to 100 µl with plain DMEM and added to the wells. After 3 h of incubation, 100 µl of DMEM with 10 % FCS was added to the cells. The medium was changed to 10 % complete medium after 24 h and the reporter gene activity was estimated after 48 h. The cells were washed twice with PBS and lysed in 50 µl of lysis buffer (0.25 M Tris.HCl, pH 8.0 and 0.5% NP40). Care was taken to ensure complete lysis. The β-galactosidase activity per well was estimated by adding 50 µl of 2X substrate solution (1.33 mg/ml of ONPG, 0.2 M sodium phosphate, pH 7.15 and 2 mM magnesium chloride) to the lysate in a 96-well plate. Absorption at 405 nm was converted to β-galactosidase units by using calibration curve constructed with pure commercial β-galactosidase enzyme. The values of β-galactosidase units in replicate plates assayed on the same day varied by less than 30 %. The transfection efficiency values reported were average values from four replicate transfection plates assayed on the same day. Each transfection experiment was repeated three times on three different days and the day-to-day variation in average transfection efficiency values for identically treated replicate transfection plates was 2-3 fold and was dependent on the cell density and conditions of the cells. Figure 1 shows representative transfection results obtained with cationic lipids **1-5**. As shown in Figure 1, the in vitro transfection efficiency of cationic lipid **2** is comparable to or better than that of DMRIE, one of the most extensively used commercially available cationic transfection lipids used in in vitro gene delivery.

### Example 7:

**Lipid:DNA Interactions Assay.** Intercalation-induced fluorescence increase and competition with cationic lipids to bind to DNA has made Ethidium Bromide (EtBr) an excellent tool to study cationic lipid-DNA interactions. To assess the representative lipid:DNA interactions for the cationic lipids of the present invention, we have titrated the EtBr: pCMV β-gal complex with increasing amounts of cationic lipids **1-5.**

The extent of Et.Br binding to the DNA was monitored by the changes in the fluorescence. EtBr fluorescence was monitored in Hitachi 4500 fluorimeter by setting the excitation wavelength at 518 nm and emission wavelength at 585 nm. To one ml of TE buffer (pH 8.0), 0.78 nmoles of DNA and 2.5 nmoles of EtBr were added. The change in fluorescence was monitored after adding small volumes of lipids **1-5** to the EtBr:DNA complex. Arbitrary fluorescence values were recorded after allowing sufficient time for equilibration. The order of addition of EtBr or lipid to DNA did not alter the final values indicating that the equilibrium does not depend on the order of addition and reaches in minutes. Percent fluorescence was calculated considering the fluorescence value in the absence of lipid as 100.

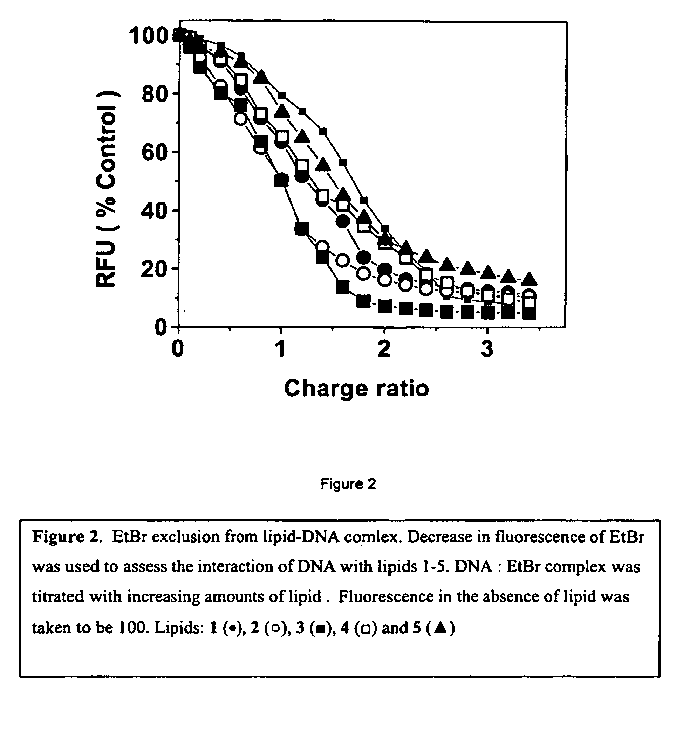

The data in Figure 2 shows that lipids **1, 4** and **5** interact poorly with DNA as seen by their relatively poor ability to exclude ethidium bromide from DNA. Lipids **2** and **3** interact with DNA equally well up to lipid:DNA ratio of 1.25 or so (Figure 2). The decrease in EtBr fluorescence at charge ratios of 3:1 was greater than 80 % with all the lipids **1-5**. Taken together the transfection results shown in Figure 1 and the lipid:DNA interaction profiles shown in Figure 2, it seems that strong lipid:DNA interactions for the presently described cationic lipids results into better intracellular gene delivery.

### Example 8:

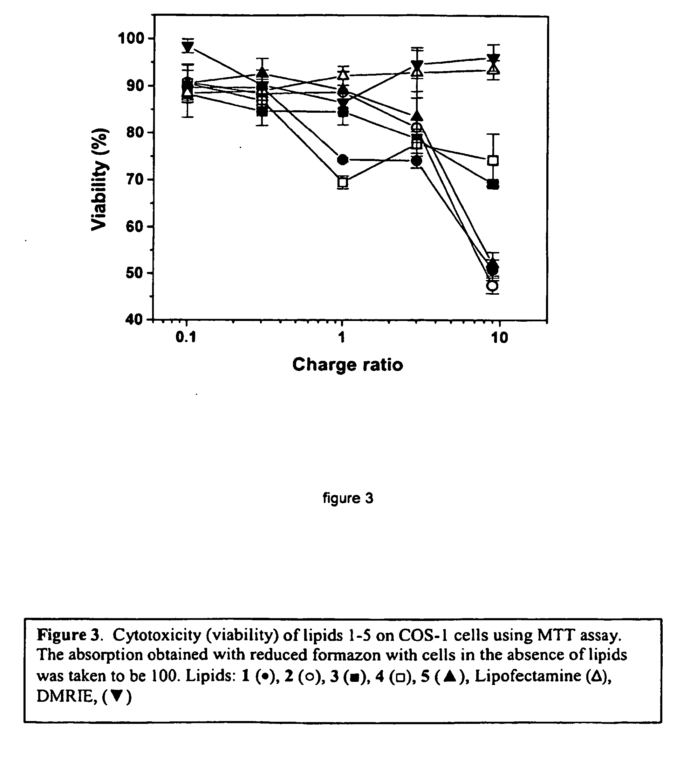

**Toxicity Assays.** An MTT based viability assay was performed to assess the cytotoxicity of lipids **1-5** at various lipid:DNA charge ratios with COS-1 cells as described previously (Banerjee, R. et al. 1999). Except lipid **1,** all the other lipids **(2-5)** showed least cytotoxicity even at a 9:1 lipid:DNA charge ratio (Figure 3). For lipids **1, 4** and **5** at a 3:1 charge ratio the toxicity varied from 15-25 % of the treated cells. The toxicity of lipid **2** (with shorter alkyl chains) may originate from its probable detergent-like cell-lysing activity. As shown in Figure 3, the cellular toxicity profiles of lipids **2-5** of the present invention are better than those of several commercially available cationic transfection lipids including lipofectin, lipofectAmine and DMRIE.

### ADVANTAGES

1. The novel cationic lipids of the present invention are particularly useful to combat genetic diseases by non-viral gene therapy.
2. The cationic amphiphiles of the present invention do not have glycerol-backbone in their molecular structures.
3. The cationic amphiphiles are useful for delivering polyanions, polypeptides and nucleopolymers into cells.

## Claims

1. A cationic amphiphile as represented by general formula (II) 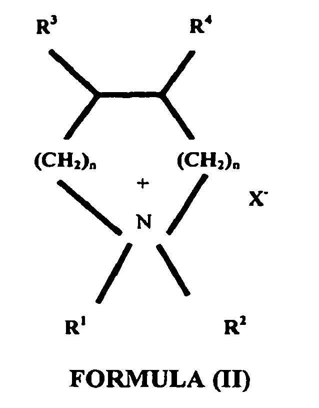
Wherein R₁, R₂, R₃ and R₄ independently or in combination represented as described below:
n=1
R₁ = R₂ = CH₃-(CH₂)₁₀CH₂-, CH₃-(CH₂)₁₂CH₂-, CH₃-(CH₂)₁₄ CH₂-, CH₃-(CH₂)₁₆ CH₂-
and/or CH₃-(CH₂)₇-CH=CH(CH₂)₇CH₂
R₃ = R₄ = hydroxyl group
X = an inorganic or organic anion.

2. A compound according to claim 1 wherein X is selected from halogen atom, tosyl group and acetate group.

3. A process for the preparation of cationic amphiphiles according to claim 1 or 2 said process comprising the steps of:
(a) coupling an appropriate saturated or unsaturated lipophilic aliphatic bromide with saturated or unsaturated lipophilic aliphatic alkyl amine in polar aprotic solvents in presence of base to obtain the corresponding aliphatic hydrophobic secondary amine;
(b) protecting both the terminal primary alcohol groups of an aliphatic alcohols containing additional polar functionalities with suitable protecting groups in polar aprotic solvent;
(c) reacting the secondary amine obtained in step (a) with the primary hydroxyl protected aliphatic polar intermediate obtained in step (b) in polar aprotic solvents in presence of an organic base to obtain quaternized amphiphile compound;
(d) passing the quaternized amphiphilic compound obtained in step (c) through anion-exchange column chromatography and eluting with a mixture of polar organic solvent to obtain the required cationic amphiphile.

4. A process as claimed in claim 3, wherein the aliphatic saturated alkyl bromide is selected from the group consisting of 10-30 carbon atoms.

5. A process as claimed in claim 3, wherein the aliphatic saturated alkyl amine is selected from the group consisting of 10-30 carbon atoms.

6. A process as claimed in claim 3, wherein the aliphatic unsaturated alkyl amine used is selected from the group consisting of 10-30 carbon atoms.

7. A process as claimed in claim 3, wherein a substituted primary hydroxyl as claimed in step (c) is selected from a group consisting of hydroxyl, hydroxy alkyl, amino or primary amino and most preferably hydroxyl or amino.

8. A process as claimed in claim 3, wherein the amino group is protected with t-boc, f-moc or any other suitable protective agents.

9. A process as claimed in claim 3, wherein the polar aprotic solvents in which the reaction is carried out are selected from the group comprising dimethyl formamide, dimethylsulphoxide, pyridine, triethyl amine.

10. A process as claimed in claim 3, wherein the reaction is carried out in the presence of weak base selected from inorganic alkali metal carbonates.

11. A process as claimed in claim 3, wherein the primary alcohol protection group is selected from tosyl chloride, mesyl chloride.

12. A process as claimed in claim 3, wherein the polar aprotic solvent used in step (a) is selected from dimethyl sulphoxide, N,N-dimethyl formamide, ethyl acetate, tetrahydrofuran.

13. A process as claimed in claim 3, wherein the reaction of step (a) is carried out at a temperature between 50°C to 100°C.

14. A process as claimed in claim 3, wherein the protection of terminal primary hydroxyl groups of step (b) is carried out at a temperature between -10° C to 50° C.

15. A process as claimed in claim 3, wherein the primary alochol is selected from erythrytol or its homologues.

16. A process as claimed in claim 3, wherein the polar aprotic solvent used in step (c) is selected from dimethyl sulphoxide, N,N-dimethyl formamide, ethyl acetate, tetrahydrofuran.

17. A process as claimed in claim 3, wherein the organic base used in step (c) is selected from triethyl amine, pyridine, piperidine.

18. A process as claimed in claim 3, wherein the reaction of step (c) is carried out at a temperature between 50° C to 100°C.

19. A process as claimed in claim 3, wherein the anion-exchange resin used in step (d) is selected from resin having chloride or bromide ion available for exchange.

20. A process as claimed in claim 3, wherein the organic solvents used as ingredients of the polar eluent in step (d) is selected from methanol, ethanol, chloroform, dichloro methane, ethyl acetate.

21. A pharmaceutical composition for intracellular delivery of biologically active molecules, said composition comprising:
- at least a cationic amphiphile used to facilitate the intracellular delivery of biologically active molecules;
- a bioactive molecule;
- a colipid; and
- optionally an additive;
The cationic amphiphile being as defined in claim 1 or 2, and :
the biologically active molecule is selected from the group consisting of ribosomal RNA, antisense polynucleotide of DNA or RNA, polynucleotide of genomic DNA, cDNA or mRNA that encodes for therapeutically important protein, nucleic acid, an oligonucleotide and a peptide.

22. The pharmaceutical composition of claim 21, wherein the colipid is selected from the group consisting of cholesterol, phosphatidylethanolamine, and phosphatidylglycerol.

23. A pharmaceutical composition according to claim 21 or 22, wherein the nucleic acid is circular, linear plasmid or RNA.

24. A pharmaceutical composition according to anyone of claims 21-23 wherein the preferred range of cationic amphiphile and colipid is in the ratio of 1:0-1:2.5.

25. A pharmaceutical composition according to anyone of claims 21-24, wherein said composition administered comprises an effective amount of DNA in the range of 0.1-0.5 µg with regard to 50.000 cells of an in vitro system.

26. A pharmaceutical composition according to anyone of claims 21-25, wherein said composition can be administered intravenously, intramuscularly and intraperitonially.

27. A pharmaceutical composition according to anyone of claims 21-26, wherein said additives are selected from physiologically acceptable additives.

28. A pharmaceutical composition according to anyone of claims 21-27, wherein said additives are used to stabilize the formulation and for the effective delivery of the biologically active molecule.

29. A pharmaceutical composition according to anyone of claims 21-28 wherein said composition can be formulated with lipophilic therapeutic anti cancer agents selected from doxorubicin, paclitaxel, docetaxel and 5-fluorouracil.

30. A pharmaceutical composition according to anyone of claims 21-29 wherein said composition is formulated with viral agents selected from Acyclovir.

31. A pharmaceutical composition according to anyone of claims 21-30, wherein said composition is formulated with antibiotics selected from amphotericin B.

32. A pharmaceutical composition according to claims 21-31, wherein said composition is formulated with an anti-influenza agent to deliver to the lung, the primary site of the infection.

33. Use of a cationic amphiphile represented by a general formula (I) 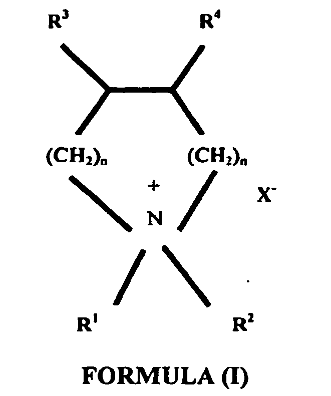 wherein;
R¹ and R² independently represent hydrogen atom or a lipophilic moiety represented by a C₈₋₂₂ saturated alkyl group or an unsaturated alkyl group having one to three unsaturation,
excluding the possible definition for R¹ and R² being simultaneously hydrogen atoms;
R³ and R⁴ are independently represented by any combination of groups selected from hydrogen, hydroxy, hydroxy alkyl, and amino or primary amine ;
n = 1, 2 or 3;
X = an inorganic or organic anion,
for manufacturing a pharmaceutical composition for intracellular delivery of biologically active molecule for administering to a subject said biologically active amphiphile.

34. The use according to claim 33, wherein R¹ is C₈₋₂₂ alkyl group saturated or unsaturated alkyl group having one to three unsaturation and R² is hydrogen atom or C₈₋₂₂ saturated or unsaturated alkyl group having one to three double unsaturation.

35. The use according to claim 33, wherein hydroxy alkyl and primary amine consists of 1-5-carbon atoms.

36. The use according to claim 33, wherein R¹ independently represents a hydrogen atom and R² is represented by C₈₋₂₂ carbon atoms selected from saturated alkyl or unsaturated alkyl chain having one to three double bonds.

37. The use according to claim 33, wherein R¹ represents C₈₋₂₂ carbon atoms selected from saturated alkyl or unsaturated alkyl chain having one to three double bonds and R2 independently represents a hydrogen atom.

38. The use according to claim 33, wherein both R¹ and R² are represented by C₈₋₂₂ carbon atoms selected from saturated alkyl or unsaturated alkyl chain having one to three double bonds.

39. Use of at least one cationic amphiphile as defined in claim 1 or 2, for manufacturing a pharmaceutical composition for intracellular delivery of biologically active molecule to a subject.

40. A use according to anyone of claims 33 to 39, wherein the subject is selected from humans and other species including murine, feline, bovine, equine and ovine or non-human primate species.

41. A use according to anyone of claims 33 to 40, wherein said composition is used to combat genetic diseases by non-viral gene therapy.

42. A use according to anyone of claims 33-41, wherein said composition can be administered intravenously, intramuscularly and intraperitonially.

43.A use according to anyone of claims 33-42, wherein cytotoxicities are minimal and the cell viability more than 80%.

44. A use according to anyone of claims 33-43, wherein said composition is used to construct cell lines for gene therapy applications in said subject.

45. A use according to anyone of claims 33-44, wherein said biologically active molecule is selected from the group consisting of ribosomal RNA, antisense polynucleotide of DNA or RNA, polynucleotide of genomic DNA, cDNA or mRNA that encodes for therapeutically important protein, nucleic acid, an oligonucleotide and a peptide.

46. A use according to claim 45, wherein said nucleic acid is circular, linear plasmid or RNA.

## Patentansprüche

1. Kationisches Amphiphil, das durch die allgemeine Formel (II) dargestellt wird 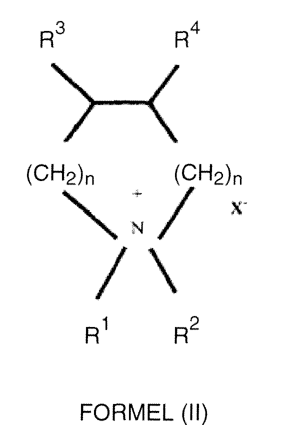
wobei R₁, R₂, R₃ und R₄ unabhängig oder in Kombination wie unten beschrieben dargestellt sind:
n=1
R₁ = R₂ = CH₃-(CH₂)₁₀CH₂-, CH₃-(CH₂)₁₂CH₂-, CH₃-(CH₂)₁₄CH₂-,
CH₃-(CH₂)₁₆CH₂- und/oder CH₃-(CH₂)₇-CH=CH(CH₂)₇CH₂
R₃ = R₄ = Hydroxylgruppe
X = ein anorganisches oder organisches Anion.

2. Verbindung nach Anspruch 1, wobei X gewählt ist aus Halogenatom, Tosylgruppe und Acetatgruppe.

3. Verfahren zur Herstellung von kationischen Amphiphilen nach Anspruch 1 oder 2, wobei das Verfahren die Schritte umfaßt von:
(a) Koppeln eines geeigneten gesättigten oder ungesättigten lipophilen aliphatischen Bromids mit einem gesättigten oder ungesättigten lipophilen aliphatischen Alkylamin in polaren aprotischen Lösungsmitteln in Gegenwart einer Base, um das entsprechende aliphatische hydrophobe sekundäre Amin zu erhalten;
(b) Schützen beider terminaler primärer Alkoholgruppen eines aliphatischen Alkohols, der zusätzliche polare Funktionalitäten enthält, mit geeigneten Schutzgruppen in einem polaren aprotischen Lösungsmittel;
(c) Reagieren des sekundären Amins, das in Schritt (a) erhalten wurde, mit dem primären hydroxylgeschützten aliphatischen polaren Intermediat, das in Schritt (b) erhalten wurde, in polaren aprotischen Lösungsmitteln in Gegenwart einer organischen Base, um eine quaternisierte amphiphile Verbindung zu erhalten;
(d) Passieren der quaternisierten amphiphilen Verbindung, die in Schritt (c) erhalten ist, durch Anionenaustauschersäulenchromatographie und Eluieren mit einem Gemisch von polarem organischem Lösungsmittel, um das geforderte kationische Amphiphil zu erhalten.

4. Verfahren nach Anspruch 3, wobei das aliphatische gesättigte Alkylbromid gewählt ist aus der Gruppe, die besteht aus 10 bis 30 Kohlenstoffatomen.

5. Verfahren nach Anspruch 3, wobei das aliphatische gesättigte Alkylamin gewählt ist aus der Gruppe, die besteht aus 10 bis 30 Kohlenstoffatomen.

6. Verfahren nach Anspruch 3, wobei das verwendete aliphatische ungesättigte Alkylamin gewählt ist aus der Gruppe, die besteht aus 10 bis 30 Kohlenstoffatomen.

7. Verfahren nach Anspruch 3, wobei ein substituiertes primäres Hydroxyl wie in Schritt (c) beansprucht, ausgewählt wird aus einer Gruppe, die besteht aus Hydroxyl, Hydroxyalkyl, Amino oder Primäramino und am Bevorzugtesten Hydroxyl oder Amino.

8. Verfahren nach Anspruch 3, wobei die Aminogruppe geschützt ist mit t-Boc, F-moc oder beliebigen anderen geeigneten Schutzreagenzien.

9. Verfahren nach Anspruch 3, wobei die polaren aprotischen Lösungsmittel, in welchen die Reaktion ausgeführt wird, gewählt sind aus der Gruppe, die Dimethylformamid, Dimethylsulphoxid, Pyridin, Triethylamin umfaßt.

10. Verfahren nach Anspruch 3, wobei die Reaktion ausgeführt wird in Gegenwart einer schwachen Base, die gewählt ist aus anorganischen Alkalimetallcarbonaten.

11. Verfahren nach Anspruch 3, wobei die primäre Alkoholschutzgruppe gewählt ist aus Tosylchlorid, Mesylchlorid.

12. Verfahren nach Anspruch 3, wobei das polare aprotische Lösungsmittel, das in Schritt (a) verwendet wird, gewählt ist aus Dimethylsulphoxid, N,N-Dimethylformamid, Ethylacetat, Tetrahydrofuran.

13. Verfahren nach Anspruch 3, wobei die Reaktion von Schritt (a) bei einer Temperatur zwischen 50°C und 100°C ausgeführt wird.

14. Verfahren nach Anspruch 3, wobei der Schutz der terminalen primären Hydroxylgruppen von Schritt (b) ausgeführt wird bei einer Temperatur zwischen -10°C bis 50°C.

15. Verfahren nach Anspruch 3, wobei der primäre Alkohol ausgewählt wird aus Erythritol und seinen Homologa.

16. Verfahren nach Anspruch 3, wobei das polare aprotische Lösungsmittel, das in Schritt (c) verwendet wird, gewählt ist aus Dimethylsulphoxid, N,N-Dimethylformamid, Ethylacetat, Tetrahydrofuran.

17. Verfahren nach Anspruch 3, wobei die in Schritt (c) verwendete organische Base gewählt ist aus Triethylamin, Pyridin, Piperidin.

18. Verfahren nach Anspruch 3, wobei die Reaktion von Schritt (c) bei einer Temperatur zwischen 50°C und 100°C ausgeführt wird.

19. Verfahren nach Anspruch 3, wobei das Anionenaustauscherharz, das in Schritt (d) verwendet wird, gewählt ist aus einem Harz, das Chlorid- oder Bromidionen zum Austausch verfügbar hat.

20. Verfahren nach Anspruch 3, wobei die organischen Lösungsmittel, die als Inhaltsstoffe des polaren Elutionsmittels in Schritt (d) verwendet sind, gewählt sind aus Methanol, Ethanol, Chloroform, Dichlormethan, Ethylacetat.

21. Pharmazeutische Zusammensetzung zur intrazellulären Abgabe von biologisch aktiven Molekülen, wobei die Zusammensetzung umfaßt:
- wenigstens ein kationisches Amphiphil, das verwendet wird, um die intrazelluläre Abgabe von biologisch aktiven Molekülen zu erleichtern;
- ein bioaktives Molekül;
- ein Co-Lipid; und
- optional einen Zusatz;
wobei das kationische Amphiphil wie in Anspruch 1 oder 2 definiert ist, und:
das biologisch aktive Molekül ausgewählt ist aus der Gruppe, die besteht aus ribosomaler RNA, Antisense-Polynukleotid von DNA oder RNA, Polynukleotid von genomischer DNA, cDNA oder mRNA, die für therapeutisch wichtiges Protein, Nukleinsäure, ein Oligonukleotid und ein Peptid codiert.

22. Pharmazeutische Zusammensetzung nach Anspruch 21, wobei das Co-Lipid gewählt ist aus der Gruppe, die besteht aus Cholesterin, Phosphatidylethanolamin und Phosphatidylglycerin.

23. Pharmazeutische Zusammensetzung nach Anspruch 21 oder 22, wobei die Nukleinsäure ringförmig, lineares Plasmid oder RNA ist.

24. Pharmazeutische Zusammensetzung nach einem der Ansprüche 21 bis 23, wobei der bevorzugte Bereich von kationischem Amphiphil und Co-Lipid im Verhältnis von 1:0 bis 1:2,5 liegt.

25. Pharmazeutische Zusammensetzung nach einem der Ansprüche 21 bis 24, wobei die verabreichte Zusammensetzung eine wirksame Menge von DNA im Bereich von 0,1 bis 0,5 µg bezüglich 50.000 Zellen einem In-vitro-System umfaßt.

26. Pharmazeutische Zusammensetzung nach einem der Ansprüche 21 bis 25, wobei die Zusammensetzung intravenös, intramuskulär und intraperitoneal verabreicht werden kann.

27. Pharmazeutische Zusammensetzung nach einem der Ansprüche 21 bis 26, wobei die Zusätze ausgewählt sind aus physiologisch verträglichen Zusätzen.

28. Pharmazeutische Zusammensetzung nach einem der Ansprüche 21 bis 27, wobei die Zusätze verwendet werden, um die Formulierung zu stabilisieren und zur effektiven Abgabe des biologisch aktiven Moleküls.

29. Pharmazeutische Zusammensetzung nach einem der Ansprüche 21 bis 28, wobei die Zusammensetzung formuliert werden kann mit lipophilen therapeutischen Mittel gegen Krebs, gewählt aus Doxorubicin, Paclitaxel, Docetaxel und 5-Fluoruracil.

30. Pharmazeutische Zusammensetzung nach einem der Ansprüche 21 bis 29, wobei die Zusammensetzung mit viralen Mitteln formuliert ist, die aus Acyclovir ausgewählt sind.

31. Pharmazeutische Zusammensetzung nach einem der Ansprüche 21 bis 30, wobei die Zusammensetzung formuliert ist mit Antibiotika, die gewählt sind aus Amphotericin B.

32. Pharmazeutische Zusammensetzung nach Ansprüchen 21 bis 31, wobei die Zusammensetzung formuliert ist mit einem Mittel gegen Influenza zur Abgabe an die Lunge, den primären Infektionsort.

33. Verwendung eines kationischen Amphiphils, das dargestellt wird durch die allgemeine Formel (I) 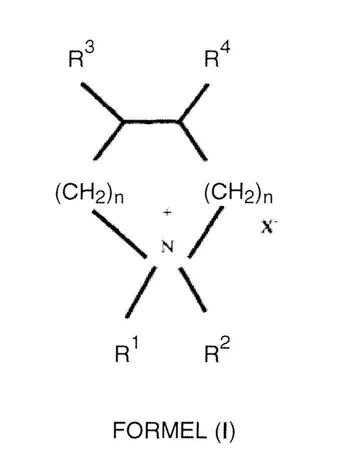 wobei:
R¹ und R² unabhängig ein Wasserstoffatom oder eine lipophile Einheit darstellt, die dargestellt wird durch eine gesättigte C₈₋₂₂-Alkylgruppe oder eine ungesättigte Alkylgruppe mit 1 bis 3 Unsättigungen,
ausgeschlossen die mögliche Definition für R¹ und R², daß sie gleichzeitig Wasserstoffatome sind;
R³ und R⁴ unabhängig dargestellt sind durch jede Kombination von Gruppen, gewählt aus Wasserstoff, Hydroxy, Hydroxyalkyl und Amino oder primäres Amin;
n = 1, 2 oder 3;
X = anorganisches oder organisches Anion,
zur Herstellung einer pharmazeutischen Zusammensetzung zur intrazellulären Abgabe von biologisch aktivem Molekül zur Verabreichung von diesem biologisch aktiven Amphiphil an ein Subjekt.

34. Verwendung nach Anspruch 33, wobei R¹ eine gesättigte oder ungesättigte C₈₋₂₂-Alkylgruppe mit 1 bis 3 Unsättigungen ist und R² ein Wasserstoffatom oder eine gesättigte oder ungesättigte C₈₋₂₂-Alkylgruppe mit 1 bis 3 Doppelunsättigungen ist.

35. Verwendung nach Anspruch 33, wobei Hydroxyalkyl und primäres Amin aus 1 bis 5 Kohlenstoffatomen bestehen.

36. Verwendung nach Anspruch 33, wobei R¹ unabhängig ein Wasserstoffatom darstellt und R² dargestellt wird durch C₈₋₂₂-Kohlenstoffatome, ausgewählt aus gesättigten Alkylketten oder ungesättigten Alkylketten mit 1 bis 3 Doppelbindungen.

37. Verwendung nach Anspruch 33, wobei R¹ C₈₋₂₂-Kohlenstoffatome darstellt, ausgewählt aus gesättigter Alkylkette und ungesättigter Alkylkette mit 1 bis 3 Doppelbindungen und R² unabhängig ein Wasserstoffatom darstellt.

38. Verwendung nach Anspruch 33, wobei sowohl R¹ als auch R² dargestellt sind durch C₈₋₂₂-Kohlenstoffatome, ausgewählt aus gesättigter Alkylkette oder ungesättigter Alkylkette mit 1 bis 3 Doppelbindungen.

39. Verwendung von wenigstens einem kationischen Amphiphil wie definiert in Anspruch 1 oder 2 zur Herstellung einer pharmazeutischen Zusammensetzung zur intrazellulären Abgabe eines biologisch aktiven Moleküls an ein Subjekt.

40. Verwendung nach einem der Ansprüche 33 bis 39, wobei das Subjekt ausgewählt ist aus Menschen oder anderen Spezies, umfassend Maus, Katze, Rind, Pferd und Schaf oder nichtmenschliche Primatenspezies.

41. Verwendung nach einem der Ansprüche 33 bis 40, wobei die Zusammensetzung verwendet wird, um genetische Krankheiten durch nichtvirale Gentherapie zu bekämpfen.

42. Verwendung nach einem der Ansprüche 33 bis 41, wobei die Zusammensetzung intravenös, intramuskulär und intraperitoneal verabreicht werden kann.

43. Verwendung nach einem der Ansprüche 33 bis 42, wobei die Zytotoxizitäten minimal sind und die Zellviabilität größer als 80% ist.

44. Verwendung nach einem der Ansprüche 33 bis 43, wobei die Zusammensetzung verwendet wird, um Zelllinien zur Gentherapie in dem Subjekt zu konstruieren.

45. Verwendung nach einem der Ansprüche 33 bis 44, wobei das biologisch aktive Molekül gewählt ist aus der Gruppe, die besteht aus ribosomaler RNA, Antisense-Polynukleotid von DNA oder RNA, Polynukleotid von genomischer DNA, cDNA oder mRNA, die für therapeutisch wichtiges Protein, Nukleinsäure, ein Oligonukleotid und ein Peptid kodiert.

46. Verwendung nach Anspruch 45, wobei die Nukleinsäure ringförmig, lineares Plasmid oder RNA ist.

## Revendications

1. Amphiphile cationique représenté par la formule générale (II) 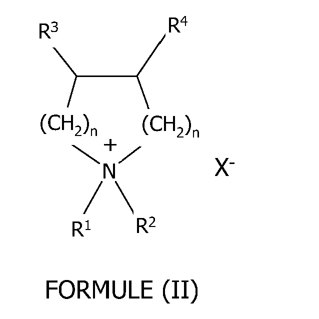 où R₁, R₂, R₃ et R₄ représentent indépendamment ou en combinaison ce qui suit:
n = 1
R₁ = R₂ = CH₃(CH₂)₁₀CH₂-, CH₃-(CH₂)₁₂CH₂-, CH₃-(CH₂)₁₄CH₂-,
CH₃(CH₂)₁₆CH₂- et/ou CH₃-(CH₂)₇-CH=CH(CH₂)₇CH₂
R₃ = R₄ = groupe hydroxyle
X = un anion inorganique ou organique.

2. Composé selon la revendication 1 où X est choisi parmi un atome d'halogène, un groupe tosyle et un groupe acétate.

3. Procédé pour la préparation d'amphiphiles cationiques selon la revendication 1 ou 2, ledit procédé comprenant les étapes de :
(a) couplage d'un bromure aliphatique lipophile saturé ou insaturé approprié avec une alkylamine aliphatique lipophile saturée ou insaturée dans des solvants aprotiques polaires en présence d'une base pour obtenir l'amine secondaire hydrophobe aliphatique correspondante ;
(b) protection des deux groupes alcool primaire terminaux d'alcools aliphatiques contenant des fonctionnalités polaires supplémentaires avec des groupes protecteurs appropriés dans un solvant aprotique polaire ;
(c) réaction de l'amine secondaire obtenue dans l'étape (a) avec l'intermédiaire polaire aliphatique à hydroxyles primaires protégés obtenu dans l'étape (b) dans des solvants aprotiques polaires en présence d'une base organique pour obtenir un composé amphiphile quaternisé ;
(d) passage du composé amphiphile quaternisé obtenu dans l'étape (c) dans une chromatographie sur colonne d'échange d'anions et élution avec un mélange de solvants organiques polaires pour obtenir l'amphiphile cationique requis.

4. Procédé selon la revendication 3 où le bromure d'alkyle saturé aliphatique est choisi dans le groupe consistant en 10-30 atomes de carbone.

5. Procédé selon la revendication 3 où l'alkylamine saturée aliphatique est choisie dans le groupe consistant en 10-30 atomes de carbone.

6. Procédé selon la revendication 3 où l'alkylamine insaturée aliphatique utilisée est choisie dans le groupe consistant en 10-30 atomes de carbone.

7. Procédé selon la revendication 3 où un hydroxyle primaire substitué selon l'étape (c) est choisi dans un groupe consistant en hydroxyle, hydroxyalkyle, amino ou amino primaire et de manière particulièrement préférable hydroxyle ou amino.

8. Procédé selon la revendication 3 où le groupe amino est protégé avec t-boc, f-moc ou tout autre agent protecteur approprié.

9. Procédé selon la revendication 3 où les solvants aprotiques polaires dans lesquels la réaction est conduite sont choisis dans le groupe comprenant le diméthylformamide, le diméthylsulfoxyde, la pyridine, la triéthylamine.

10. Procédé selon la revendication 3 où la réaction est conduite en présence d'une base faible choisie parmi les carbonates de métaux alcalins inorganiques.

11. Procédé selon la revendication 3 où le groupe de protection d'alcool primaire est choisi parmi le chlorure de tosyle, le chlorure de mésyle.

12. Procédé selon la revendication 3 où le solvant aprotique polaire utilisé dans l'étape (a) est choisi parmi le diméthylsulfoxyde, le N,N-diméthylformamide, l'acétate d'éthyle, le tétrahydrofurane.

13. Procédé selon la revendication 3 où la réaction de l'étape
(a) est conduite à une température entre 50°C et 100°C.

14. Procédé selon la revendication 3 où la protection de groupes hydroxyle primaires terminaux de l'étape (b) est conduite à une température entre -10°C et 50°C.

15. Procédé selon la revendication 3 où l'alcool primaire est choisi parmi l'érythritol ou ses homologues.

16. Procédé selon la revendication 3 où le solvant aprotique polaire utilisé dans l'étape (c) est choisi parmi le diméthylsulfoxyde, le N,N-diméthylformamide, l'acétate d'éthyle, le tétrahydrofurane.

17. Procédé selon la revendication 3 où la base organique utilisée dans l'étape (c) est choisie parmi la triéthylamine, la pyridine, la pipéridine.

18. Procédé selon la revendication 3 où la réaction de l'étape (c) est conduite à une température entre 50°C et 100°C.

19. Procédé selon la revendication 3 où la résine d'échange d'anions utilisée dans l'étape (d) est choisie parmi une résine ayant des ions chlorure ou bromure disponibles pour l'échange.

20. Procédé selon la revendication 3 où les solvants organiques utilisés comme ingrédients de l'éluant polaire dans l'étape (d) sont choisis parmi le méthanol, l'éthanol, le chloroforme, le dichlorométhane, l'acétate d'éthyle.

21. Composition pharmaceutique pour la délivrance intracellulaire de molécules biologiquement actives, ladite composition comprenant :
- au moins un amphiphile cationique utilisé pour faciliter la délivrance intracellulaire de molécules biologiquement actives ;
- une molécule bioactive ;
- un colipide ; et
- éventuellement un additif ;
l'amphiphile cationique étant tel que défini dans la revendication 1 ou 2, et :
la molécule biologiquement active est choisie dans le groupe consistant en l'ARN ribosomique, un polynucléotide antisens d'ADN ou d'ARN, un polynucléotide d'ADN génomique, d'ADNc ou d'ARNm qui code une protéine thérapeutiquement importante, un acide nucléique, un oligonucléotide et un peptide.

22. Composition pharmaceutique selon la revendication 21 où le colipide est choisi dans le groupe consistant en le cholestérol, la phosphatidyléthanolamine et le phosphatidylglycérol.

23. Composition pharmaceutique selon la revendication 21 ou 22 où l'acide nucléique est un plasmide linéaire, circulaire, ou un ARN.

24. Composition pharmaceutique selon l'une quelconque des revendications 21-23, où la plage préférée d'amphiphile cationique et de colipide est dans le rapport de 1:0 - 1:2,5.

25. Composition pharmaceutique selon l'une quelconque des revendications 21-24 où ladite composition administrée comprend une quantité efficace d'ADN dans la plage de 0,1-0,5 µg pour 50 000 cellules d'un système in vitro.

26. Composition pharmaceutique selon l'une quelconque des revendications 21-25 où ladite composition peut être administrée par voie intraveineuse, intramusculaire et intrapéritonéale.

27. Composition pharmaceutique selon l'une quelconque des revendications 21-26 où lesdits additifs sont choisis parmi les additifs physiologiquement acceptables.

28. Composition pharmaceutique selon l'une quelconque des revendications 21-27 où lesdits additifs sont utilisés pour stabiliser la formulation et pour la délivrance efficace de la molécule biologiquement active.

29. Composition pharmaceutique selon l'une quelconque des revendications 21-28 où ladite composition peut être formulée avec des agents anticancéreux thérapeutiques lipophiles choisis parmi la doxorubicine, la paclitaxel, le docétaxel et le 5-fluorouracile.

30. Composition pharmaceutique selon l'une quelconque des revendications 21-29 où ladite composition est formulée avec des agents viraux choisis parmi l'acyclovir.

31. Composition pharmaceutique selon l'une quelconque des revendications 21-30 où ladite composition est formulée avec des antibiotiques choisis parmi l'amphotéricine B.

32. Composition pharmaceutique selon les revendications 21-31 où ladite composition est formulée avec un agent antigrippal pour la délivrance aux poumons, le site primaire de l'infection.

33. Utilisation d'un amphiphile cationique représenté par la formule générale (I) 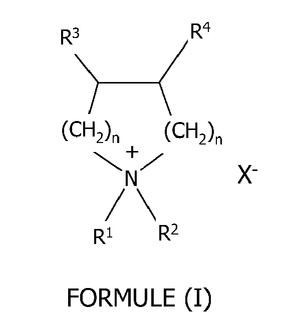 où :
R¹ et R² représentent indépendamment un atome d'hydrogène ou un groupement lipophile représenté par un groupe C₈₋₂₂ alkyle saturé ou un groupe alkyle insaturé ayant 1 à 3 insaturations, à l'exclusion de la définition possible de R¹ et R² étant simultanément des atomes d'hydrogène ;
R³ et R⁴ sont représentés indépendamment par toute combinaison de groupes choisis parmi hydrogène, hydroxy, hydroxyalkyle et amino ou amine primaire;
n = 1, 2 ou 3 ;
X = un anion inorganique ou organique,
pour la fabrication d'une composition pharmaceutique pour la délivrance intracellulaire d'une molécule biologiquement active pour l'administration à un sujet dudit amphiphile biologiquement actif.

34. Utilisation selon la revendication 33 où R¹ est un groupe C₈₋₂₂ alkyle saturé ou un groupe alkyle insaturé ayant une à trois insaturations et R² est un atome d'hydrogène ou un groupe C₈₋₂₂ alkyle saturé ou insaturé ayant une à trois doubles insaturations.

35. Utilisation selon la revendication 33 où l'hydroxyalkyle et l'amine primaire consistent en 1-5 atomes de carbone.

36. Utilisation selon la revendication 33 où R¹ représente indépendamment un atome d'hydrogène et R² est représenté par C₈₋₂₂ atomes de carbone choisis parmi une chaîne alkyle saturée ou une chaîne alkyle insaturée ayant une à trois doubles liaisons.

37. Utilisation selon la revendication 33 où R¹ représente C₈₋₂₂ atomes de carbone choisis parmi une chaîne alkyle saturée ou une chaîne alkyle insaturée ayant 1 à 3 doubles liaisons et R² représente indépendamment un atome d'hydrogène.

38. Utilisation selon la revendication 33 où R¹ et R² sont représentés l'un et l'autre par C₈₋₂₂ atomes de carbone choisis parmi une chaîne alkyle saturée ou une chaîne alkyle insaturée ayant 1 à 3 doubles liaisons.

39. Utilisation d'au moins un amphiphile cationique selon la revendication 1 ou 2 pour la fabrication d'une composition pharmaceutique pour la délivrance intracellulaire d'une molécule biologiquement active à un sujet.

40. Utilisation selon l'une quelconque des revendications 33 à 39 où le sujet est choisi parmi les humains et d'autres espèces incluant les espèces murines, félines, bovines, équines et ovines ou les espèces de primates non-humains.

41. Utilisation selon l'une quelconque des revendications 33-40 où ladite composition est utilisée pour combattre les maladies génétiques par une thérapie génique non virale.

42. Utilisation selon l'une quelconque des revendications 33-41 où ladite composition peut être administrée par voie intraveineuse, intramusculaire et intrapéritonéale.

43. Utilisation selon l'une quelconque des revendications 33-42 où les cytotoxicités sont minimales et la viabilité cellulaire est supérieure à 80 %.

44. Utilisation selon l'une quelconque des revendications 33-43 où ladite composition est utilisée pour construire des lignées cellulaires pour des applications de thérapie génique chez ledit sujet.

45. Utilisation selon l'une quelconque des revendications 33-44 où ladite molécule biologiquement active est choisie dans le groupe consistant en l'ARN ribosomique, un polynucléotide antisens d'ADN ou d'ARN, un polynucléotide d'ADN génomique, d'ADNc ou d'ARNm qui code une protéine thérapeutiquement importante, un acide nucléique, un oligonucléotide et un peptide.

46. Utilisation selon la revendication 45 où ledit acide nucléique est un plasmide linéaire, circulaire ou un ARN.
